(19) Europäisches Patentamt
European Patent Office
Office européen des brevets

(11) Numéro de publication: **0 225 826**
**B1**

(12) # FASCICULE DE BREVET EUROPEEN

(45) Date de publication du fascicule du brevet: **20.06.90**

(21) Numéro de dépôt: **86402600.0**

(22) Date de dépôt: **21.11.86**

(51) Int. Cl.⁵: **C07C 271/08**, C07C 333/02, C07C 311/01, C08F 20/36, C14C 11/00

(54) **Monomères acryliques fluorés, polymères en dérivant et leur application comme agents hydrophobes et oléophobes.**

(30) Priorité: **03.12.85 FR 8517882**

(43) Date de publication de la demande: **16.06.87 Bulletin 87/25**

(45) Mention de la délivrance du brevet: **20.06.90 Bulletin 90/25**

(84) Etats contractants désignés: **AT BE CH DE FR GB IT LI LU NL SE**

(56) Documents cités: **EP-A- 0 040 923**

(73) Titulaire: **ATOCHEM, 4 & 8, Cours Michelet La Défense 10, F-92800 Puteaux(FR)**

(72) Inventeur: **Lina, Marie-José, Résidence "Les Jonquières" 6, rue Boyer, F-69160 Tassin La Demi Lune(FR)**
Inventeur: **Dessaint, André, 17 B, rue des Racques Boulincourt, F-60600 Clermont(FR)**

(74) Mandataire: **Leboulenger, Jean et al, ATOCHEM Département Propriété Industrielle, F-92091 Paris la Défense 10 Cédex 42(FR)**

**EP 0 225 826 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne le domaine des produits fluorés destinés au traitement hydrofuge et oléofuge de substrats tels que textiles, tapis-moquettes, revêtements muraux, bois, matériaux de construction, métaux, matières plastiques, et a notamment pour objet des produits utilisables plus particulièrement pour la protection du cuir dont la finition et l'entretien doivent présenter les caractéristiques suivantes : souplesse, aspect et toucher agréables.

L'utilisation de résines acryliques fluorées dans ces applications est bien connue, mais présente un certain nombre d'inconvénients : toucher légèrement collant, mauvaise résistance aux nettoyages et à l'abrasion, légère modification de l'aspect du support.

Des compositions comprenant des groupes perfluorés et des enchaînements uréthannes ont déjà été proposées ; voir par exemple les brevets U.S. 3 468 924, 3 503 915, 3 528 849, 3 896 035, 3 896 251 et 4 024 178, FR 2 062 244, DE 1620 965, CA 1 071 225, EP 103752, CH 520 813 et 512 624. Malheureusement, ces produits ne donnent pas toujours satisfaction soit parce que la synthèse des intermédiaires est difficile, soit parce qu'ils doivent être associés à des copolymères acryliques car ils ne sont pas filmogènes, ne résistent pas au nettoyage à sec et/ou ne présentent pas de bonnes propriétés antitaches, soit encore parce qu'ils doivent être présentés en émulsion aqueuse en raison de leur faible solubilité dans les solvants.

Pour empêcher le transfert de la poudre d'impression lors de la préparation de transparents par un procédé électrographique, on a proposé dans le brevet EP 0 100 277 l'emploi de polymères fluorés à groupements uréthanne. Sont notamment décrits dans ce brevet des polymères dérivés du diuréthanne fluoré de formule :

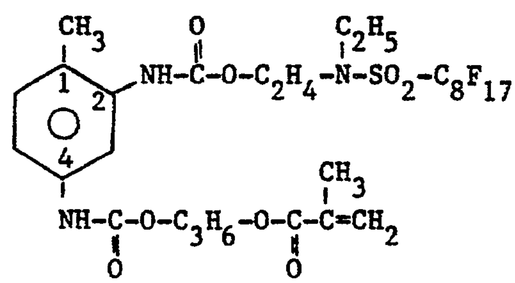

(I)

Cependant, les polymères obtenus à partir du diuréthanne fluoré (I) présentent l'inconvénient de former des gels insolubles ou de fournir des performances hydrophobes et oléophobes insuffisantes.

Il a maintenant été trouvé, de façon tout-à-fait inattendue, qu'on peut remédier à cet inconvénient en utilisant un uréthanne fluoré dans lequel la chaîne polyfluorée est fixée en position 4 et non pas en position 2 comme dans le composé de formule (I) selon le brevet EP 0 100 227. Les polymères dérivés de ces diuréthannes à chaîne polyfluorée en position 4 sont bien solubles dans les solvants usuels; ils présentent d'excellentes propriétés hydrophobes et oléophobes et sont en particulier parfaitement adaptés au traitement du cuir.

La présente invention a donc d'abord pour objet, en tant que monomères acryliques fluorés, les diuréthannes de formule générale:

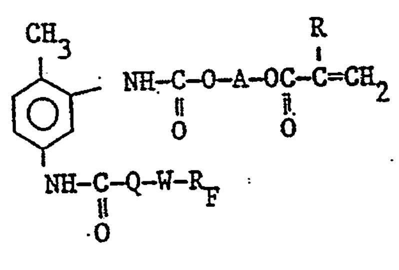

(II)

dans laquelle:

$R_F$ représente un radical perfluoroalkyle à chaîne droite ou ramifiée contenant de 2 à 20 atomes de carbone (de préférence 4 à 16),

R représente un atome d'hydrogène ou, de préférence, un radical méthyle,

A représente un enchaînement bivalent de 2 à 9 atomes de carbone, pouvant comporter un ou plusieurs atomes d'oxygène,

W-Q représente un enchaînement bivalent choisi parmi ceux de formules:

$-(CH_2)_p-O-$

$-SO_2\underset{R'}{N}-(CH_2)_q-O-$

$-(CH_2)_p-SO_2\underset{R'}{N}-(CH_2)_q-O-$

$-(CH_2)_p-O-(CH_2)_q-O-$

$-(CH_2)_p-S-(CH_2)_q-O-$

$-(CH_2)_p-(OCH_2CH_2)_q-O-$

$-(CH_2)_p-SO_2-(CH_2)_q-O-$

$-\underset{\underset{O}{\parallel}}{C}-\underset{R'}{N}-(CH_2)_p-O-$

$-\underset{\underset{O}{\parallel}}{C}-O-(CH_2)_p-O-$

$-CH=CH-(CH_2)_p-O-$

$-(CH_2)_pS-$

$-(CH_2)_pNH-$

$-SO_2-\underset{R'}{N}-(CH_2)_qNH-$

$-SO_2-\underset{R'}{N}-(CH_2)_q-\underset{CH_3}{N}-$

R' désigne un atome d'hydrogène ou un radical alkyle contenant 1 à 4 atomes de carbone, p et q, identiques ou différents, représentent chacun un nombre entier allant de 1 à 20, et de préférence égal à 2 ou 4.

Les monomères acryliques fluorés de formule (II) selon l'invention peuvent être préparés en faisant réagir dans un premier temps de toluène-diisocyanate-2,4 avec une quantité sensiblement équimolaire d'un composé polyfluoré de formule :

$R_F-W-Q-H$ (III)

pour former un isocyanate-uréthanne fluoré de formule :

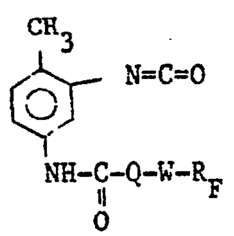

(IV)

puis en faisant réagir cet isocyanate-uréthanne avec une quantité sensiblement équimolaire d'un ester acrylique de formule :

$$HO-A-O-\underset{\substack{\|\\ O}}{C}-\overset{\substack{R\\ |}}{C}=CH_2 \qquad (V)$$

Le composé polyfluoré (III) est un composé contenant un atome d'hydrogène mobile sous forme d'un groupement terminal hydroxyle, thiol ou amino primaire ou secondaire, lié au radical perfluoroalkyle par un pont alkylène directement ou via un groupement sulfonamido, carbonamido, éther, thioéther, sulfonyle ou ester carboxylique.
Comme exemples de tels composés polyfluorés, on peut citer plus particulièrement ceux de formules :

$$R_F-(CH_2)_p-OH \qquad (III-a)$$

$$R_F-SO_2\underset{\substack{|\\ R}}{N}-(CH_2)_q-OH \qquad (III-b)$$

$$R_F-(CH_2)_p-SO_2\underset{\substack{|\\ R}}{N}-(CH_2)_q-OH \qquad (III-c)$$

$$R_F-(CH_2)_p-O-(CH_2)_q-OH \qquad (III-d)$$

$$R_F-(CH_2)_p-S-(CH_2)_q-OH \qquad (III-e)$$

$$R_F-(CH_2)_p-(OCH_2CH_2)_q-OH \qquad (III-f)$$

$$R_F-(CH_2)_p-SO_2-(CH_2)_q-OH \qquad (III-g)$$

$$R_F-\underset{\substack{\|\\ O}}{C}-\overset{\substack{R\\ |}}{N}-(CH_2)_p-OH \qquad (III-h)$$

$$R_F-\underset{\substack{\|\\ O}}{C}-O-(CH_2)_p-OH \qquad (III-i)$$

$$R_F-CH=CH-(CH_2)_p-OH \qquad (III-j)$$

$$R_F-(CH_2)_p-SH \qquad (III-k)$$

$$R_F-(CH_2)_p-NH_2 \qquad (III-l)$$

$$R_F-SO_2-\underset{\substack{|\\ R}}{N}-(CH_2)_q-NH_2 \qquad (III-m)$$

$$R_F-SO_2-\underset{\substack{|\\ R'}}{N}-(CH_2)_q-N\begin{matrix}\diagup CH_3\\ \diagdown H\end{matrix} \qquad (III-n)$$

dans lesquelles $R_F$ et R" ont les mêmes significations que ci-dessus, les symboles p et q, identiques ou différents, représentent chacun un nombre entier allant de 1 à 20 et de préférence égal à 2 ou 4. Pour des raisons économiques et pratiques, il est particulièrement intéressant d'utiliser un mélange de composés représentant des radicaux $R_F$ différents.

Parmi les composés (III) on préfére ceux de formules (III-a), (III-c), (III-k), dans lesquels p et q sont égaux à 2.

Comme exemples d'esters de formule (V), on peut citer plus particulièrement les monoacrylates et monométhacrylates de diols ou de polyalkylèneglycols, tels que l'éthylèneglycol, le propylèneglycol, le propanediol-1,3, les butanediols, le phénoxy-3 propanediol-1,2, le triéthylèneglycol. On utilise de préférence le monométhacrylate d'éthylèneglycol.

La synthèse des monomères acryliques fluorés (II) selon l'invention peut être effectuée dans un solvant organique tel que les solvants cétoniques comme la méthyl éthyl cétone ou la méthyl isobutyl cétone, des esters comme l'acétate d'éthyle ou l'acétate de butyle, des solvants aromatiques comme le toluène, des alcanes comme l'hexane, l'heptane ou le cyclohexane, des éthers comme le diisopropyl éther ou le tétrahydrofuranne, des solvants halogénés comme le trichloro-1,1,1 éthane ou le trichlorotrifluoroéthane, ainsi que le diméthyl formamide et la N-méthyl-pyrrolidone.

Les réactions d'addition du composé polyfluoré $R_F$-W-Q-H et de l'ester acrylique (V) sur les groupements -N=C=O s'effectuent entre 30 et 90°C sous atmosphère inerte, par exemple sous azote anhydre. L'addition du composé polyfluoré étant lente, il est préférable d'opérer en présence d'un catalyseur tel que, par exemple, une aminetertiaire comme la triéthylamine, la triéthylènediamine et la N- méthyl-morpholine, un sel d'étain comme le dibutyl dilaurate d'étain et l'octoate d'étain, ou un sel de plomb comme le naphténate de plomb, ce catalyseur étant utilisé à raison de 0,05 à 1 % par rapport au poids total des réactifs et introduit avec l'un et/ou l'autre des réactifs.

Afin de limiter la formation concomitante de produits de diadditions symétriques, c'est-à-dire des produits de formules :

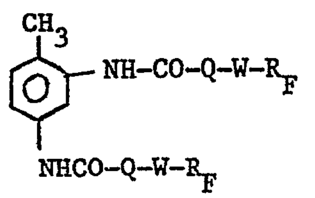

(VI)

et

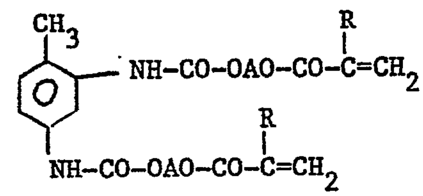

(VII)

il est préférable d'ajouter le composé polyfluoré (III) lentement, dans des conditions de dilution et de température telles que la réaction soit quasi instantanée et que l'on soit toujours en défaut de composé (III) par rapport au toluènediisocyanate. Bien qu'on ne puisse pas éviter complètement la formation de produits de diadditions symétriques, la présence de ces produits dans les solutions de monomères acryliques (II), destinées à la polymérisation, n'est pas gênante. Il est cependant possible, si on le désire, de les éliminer par cristallisation fractionnée et filtration car ils sont moins solubles dans les solvants que les monomères (II).

Au lieu d'utiliser du toluène-diisocyanate-2,4 pur, trés onéreux, il est économiquement avantageux d'employer un toluène-diisocyanate technique qui peut contenir jusqu'à environ 35 % en poids (de préférence jusqu'à environ 20) d'isomère-2,6. En effet, dans la mesure où leur proportion reste relativement faible, la présence des produits d'addition sur cet isomère-2,6 ne présente pas d'inconvénients pour les applications considérées.

L'invention a également pour objet les polymères contenant des motifs de formule :

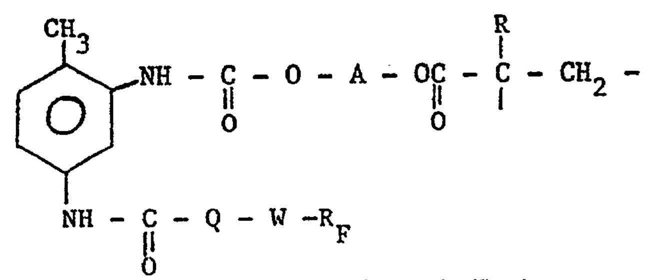

(VIII)

dans laquelle les différents symboles ont les mêmes significations. que précédemment. Ces polymères peuvent être obtenus à partir des monomères de formule (II) par homopolymérisation ou par copolymérisation avec d'autres monomères, fluorés ou non, en une proportion allant jusqu'à 90 % en poids (de préférence jusqu'à environ 50 %) par rapport au poids total de monomères.

Comme exemples de comonomères utilisables dans le cadre de la présente invention, on peut citer :

- les hydrocarbures oléfiniques inférieurs, halogénés ou non, tels que l'éthylène, le propylène, l'isobutène, le chloro-3 isobutène-1, le butadiène, l'isoprène, les chloro- et dichloro-butadiènes, les fluoro- et difluoro-butadiènes, le diméthyl-2,5 hexadiène-1,5, le diisobutylène ;
- les halogénures de vinyle, d'allyle ou de vinylidène tels que le chlorure de vinyle ou de vinylidène, le fluorure de vinyle ou de vinylidène, le bromure d'allyle, le chlorure de méthallyle ;
- le styrène et ses dérivés, tels que le vinyl-toluène, l' α- méthyl-styrène, l' α-cyanométhyl-styrène, le divinyl-benzène, le N-vinyl carbazole ;
- les esters vinyliques tels que l'acétate de vinyle, le propionate de vinyle, les esters vinyliques des acides connus sur le marché sous le nom de "Versatic acids", l'isobutyrate de vinyle, le sénécioate de vinyle, le succinate de vinyle, l'isodécanoate de vinyle, le stéarate de vinyle, le carbonate de divinyle ;
- les esters d'allyle comme l'acétate d'allyle et l'heptanoate d'allyle ;

- les éthers alkyl-vinyliques ou alkyl-allyliques, halogénés ou non, tels que le cétyl vinyl éther, le dodécyl vinyl éther, l'isobutyl vinyl éther, l'éthyl vinyl éther, le chloro-2 éthyl vinyl éther, le tétra allyloxy éthane ;
- les vinyl alkyl cétones comme la vinyl méthyl cétone ;
- les acides insaturés, tels que les acides acrylique, méthacrylique, α-chloro-acrylique, crotonique, maléique, fumarique, itaconique, citraconique et sénécioïque, leurs anhydrides et leurs esters comme les acrylates et méthacrylates de vinyle, d'allyle, de méthyle, de butyle, d'isobutyle, d'hexyle, d'heptyle, d'éthyl-2 hexyle, de cyclohexyle, de lauryle, de stéaryle ou de cellosolve, le maléate de diméthyle, le crotonate d'éthyle, le maléate acide de méthyle, l'itaconate acide de butyle, les diacrylates et diméthacrylates de glycol ou de polyalkylène glycol, comme le diméthacrylate d'éthylène glycol ou de triéthylène glycol, les acrylates et méthacrylates de dichloro-phosphato alkyle comme le méthacrylate de dichlorophosphato éthyle, ainsi que le phosphate acide de bis (méthacryloyloxy éthyle) et le méthacryloyloxypropyl-triméthoxysilane;
– l'acrylonitrile, le méthacrylonitrile, le chloro-2 acrylonitrile, l'acrylate de cyano-2 éthyle, le méthylène glutaronitrile, le cyanure de vinylidène, les cyanoacrylates d'alkyle comme le cyanoacrylate d'isopropyle, la trisacryloyl hexahydro-s-triazine, le vinyl trichlorosilane, le vinyl triméthoxysilane, le vinyl triéthoxysilane, la N-vinyl-pyrrolidone-2;
– l'alcool allylique, l'allyl glycolate, l'isobutènediol, l'allyloxy éthanol, l'o. allyl phénol, le divinyl carbinol, le glycérol-allyléther, l'acrylamide, le méthacrylamide, les maléamide et maléïmide, le N-(cyano-éthyl)-acrylamide, le N-isopropyl-acrylamide, le diacétone-acrylamide, les N-(hydroxy-méthyl) acrylamide ét méthacrylamide, les N-(alcoxyméthyl) acrylamides et méthacrylamides, le glyoxal bis-acrylamide, l'acrylate ou méthacrylate de sodium, l'acrylate de sulfo-2 éthyle, les acides vinyl-sulfonique et styrène-p-sulfonique et leurs sels alcalins, l'amino-3 crotononitrile, la monoallyl amine, les vinyl-pyridines, l'acrylate ou méthacrylate de glycidyle, l'allyl glycidyl éther, l'acroléine, le méthacrylate de N,N-diméthylaminoéthyle ou de N-tertiobutylamino-éthyle;
– les esters fluorés insaturés de formule générale:

$$R_F - W - O - \underset{\underset{O}{\|}}{C} - \overset{\overset{R}{|}}{C} = CH - R \qquad (IX)$$

dans laquelle W représente un enchaînement bivalent choisi parmi ceux de formules:

$$-(CH_2)_p-$$

$$-SO_2\underset{\underset{R'}{|}}{N}-(CH_2)_q-$$

$$-(CH_2)_p-SO_2\underset{\underset{R'}{|}}{N}-(CH_2)_q-$$

$$-(CH_2)_p-O-(CH_2)_q-$$

$$-(CH_2)_p-S-(CH_2)_q-$$

$$-(CH_2)_p-(OCH_2CH_2)_q-$$

$$-(CH_2)_p-SO_2-(CH_2)_q-$$

$$-\underset{\underset{O}{\|}}{C}-\underset{\underset{R'}{|}}{N}-(CH_2)_p-$$

$$-\underset{\underset{O}{\|}}{C}-O-(CH_2)_p-$$

$$-CH=CH-(CH_2)_p-$$

les symboles $R_F$, R, R', p et q ayant les mêmes significations que précédemment. Ces comonomères peuvent être préparés selon des procédés connus, par exemple par esterification des alcools polyfluorés correspondants de formule:

$R_F$ - W - OH (X)

au moyen d'un acide alcène-monocarboxylique de formule:

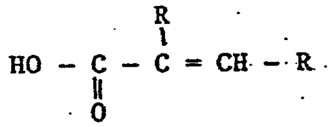 (XI)

tel que, par exemple, l'acide acrylique, l'acide méthacrylique ou l'acide crotonique, en présence d'un catalyseur comme l'acide sulfurique ou l'acide p-toluènesulfonique. Au lieu des acides de formule (XI), on peut également utiliser leurs esters, anhydrides ou halogénures. Comme exemples d'alcools polyfluorés de formule (X), on peut citer plus particulièrement ceux de formules (III-a) à (III-j) ci-dessus.

A titre de comonomères utilisables dans le cadre de la présente invention, on peut encore mentionner :

- les produits de formule (VII) ci-dessus ;
- les esters insaturés de formule :

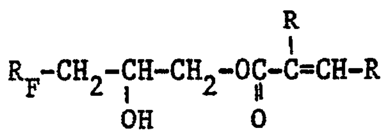 (XII)

obtenus par condensation d'un époxyde fluoré :

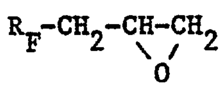 (XIII)

sur un acide alcène-monocarboxylique de formule (XI) ;

- les acrylates et méthacrylates d'éthers de polyéthylène glycols ou de polypropylène glycols de formule:

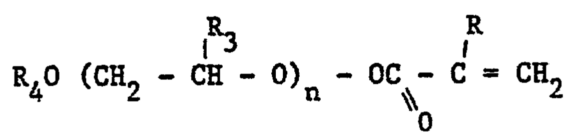 (XIV)

dans laquelle $R_3$ représente un atome d'hydrogène ou un radical méthyle, $R_4$ représente un radical alkyle et n est un nombre entier compris entre 2 et 10 ; et

- les composés de formule :

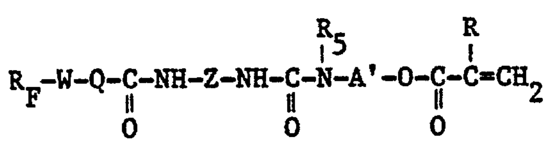 (XV)

dans laquelle les symboles R, $R_F$ et WQ ont les mêmes significations que précédemment, A' représente un groupe alkylène de 2 ou 3 atomes de carbone, $R_5$ représente un radical alkyle, cycloalkyle ou pipérazinyle, et Z représente un enchaînement bivalent aliphatique, cycloaliphatique ou aromatique. Ces composés qui font l'objet de la demande de brevet français nº 85 15347 déposée le 16 Octobre 1985 par la Demanderesse, peuvent être préparés en faisant réagir sur un diisocyanate aliphatique, cycloaliphatique ou aromatique des quantités sensiblement équimolaires d'un composé polyfluoré de formule (III) et d'un ester acrylique à groupement amino secondaire de formule :

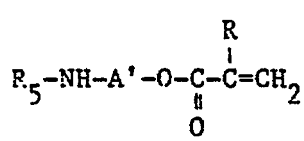 (XVI)

dans des conditions opératoires analogues à celles décrites précédemment pour la préparation des monomères (II).

Parmi les comonomères précités, on préfère plus particulièrement les acrylates et méthacrylates d'alkyle simples ou fonctionnalisés par un groupe hydroxyle, amino ou acide sulfonique, les composés fluorés (IX) et (XV), les méthacrylates d'éthers de polyéthylèneglycols, les éthers vinyliques, les chlorure et fluorure de vinyle ou de vinylidène, la vinylpyrrolidone, l'acrylamide et ses dérivés, l'acide acrylique ou méthacrylique.

Les polymères fluorés selon l'invention peuvent être préparés de façon connue en soi par polymérisation dans un solvant organique ou en émulsion aqueuse, à une température qui peut aller de la température ambiante jusqu'à point d'ébullition du milieu réactionnel. On opère de préférence entre 70 et 100°C. La concentration totale des monomères peut varier de 5 à 60 % en poids.

La polymérisation en milieu solvant peut être réalisée dans des solvants cétoniques (par exemple l'acétone, la méthyl éthyl cétone, la méthyl isobutyl cétone), des alcools (par exemple l'isopropanol), des esters (par exemple l'acétate d'éthyle ou l'acétate de butyl), des éthers (par exemple le diisopropyl éther, l'éther éthylique ou méthylique de l'éthylène glycol, le tétrahydrofuranne, le dioxanne), des hydrocarbures aliphatiques ou aromatiques, des hydrocarbures halogénés (par exemple le perchloréthylène, le trichloro-1,1,1 éthane, le trichlorotrifluoroéthane), le diméthylformamide ou la N-méthyl-pyrrolidone-2.

On effectue la polymérisation en présence d'initiateur(s) qu'on utilisé à raison de 0,1 à 1,5 % par rapport au poids total des monomères engagés. Comme initiateurs on peut utiliser des peroxydes tels que, par exemple, le peroxyde de benzoyle, le peroxyde de lauroyle, le peroxyde de succinyle et le perpivalate de tertiobutyle, ou des composés azoïques tels que, par exemple, l'azo-2,2' bis-isobutyronitrile, 1'l'azo-4,4' bis-(cyano-4 pentanoïque) et l'azodicarbonamide. On peut également opérer en présence de rayonnements U.V. et de photo-initiateurs tels que la benzophénone, la méthyl-2 anthraquinone ou le chloro-2 thioxanthone. La longueur des chaînes polymériques peut, si on le désire, être réglée à l'aide d'agents de transfert de chaînes tels que les alkyl-mercaptans, le tétrachlorure de carbone ou le triphénylméthane, utilisés à raison de 0,05 à 0,5 % par rapport au poids total de monomères.

La polymérisation en émulsion aqueuse peut être réalisée selon les techniques bien connues, en discontinu ou en continu. Les agents tensio-actifs à utiliser pour la mise en émulsion peuvent être cationiques, anioniques ou non-ioniques selon l'ionicité désirée pour le latex final, et sont de préférence choisis parmi les meilleurs émulsionnants huile-dans-eau, les moins mouillants possibles. On utilise de préférence des couples de tensio-actifs cationique / non-ionique ou anionique/non-ionique. Comme exemples d'agents tensio-actifs utilisables on peut citer plus particulièrement :

- dans la série cationique, les sels d'amines tertiaires à longue chaîne comme l'acétate de N,N-diméthyl octadécylamine, et les sels d'ammonium quaternaires d'amines grasses comme le bromure de triméthyl cétyl ammonium ou le chlorure de triméthyl dodécyl ammonium ;
- dans le série anionique, les sels alcalins d'acides alkylsulfoniques à longue chaîne et les arylalkylsulfonates alcalins ;
- dans la série non-ionique, les produits de condensation de l'oxyde d'éthylène avec les alcools gras où les alkylphénols. Il peut également être interessant d'utiliser des agents tensio-actifs à chaîne hydrophobe perfluorée tels que, par exemple, le perfluoro-octanoate d'ammonium ou le N-perfluorooctyl-sulfonyl N-éthyl aminoacétate de potassium.

Pour faciliter la mise en émulsion des monomères, il est généralement nécessaire d'utiliser des solvants organiques tels que, par exemple, des cétones (acétone, méthyl, éthyl, cétone, méthyl isobutyl cétone), des glycols ou éthers d'éthylèneglycol, des alcools (méthanol, éthanol, isopropanol), ou des mélanges de ces solvants. La quantité de solvant ne doit généralement pas dépasser le poids total des monomères.

Comme initiateurs de polymérisation en émulsion aqueuse, on peut utiliser des produits solubles dans l'eau tels que les peroxydes minéraux (par exemple l'eau oxygénée) et les persels (par exemple le persulfate de potassium), ou des initiateurs insolubles dans l'eau comme les peroxydes organiques et les composés azoïques mentionnés précédemment.

Les polymères fluorés selon l'invention peuvent également être préparés par greffage d'un isocyanate-uréthanne fluoré de formule (IV) sur un polymère acrylique à groupes OH pendants, obtenu par homopolymérisation d'un ester acrylique de formule (V) ou par copolymérisation d'un tel ester avec un ou plusieurs des comonomères précédemment cités. L'opération de greffage peut être réalisée dans les mêmes conditions que l'addition de l'ester (V) sur l'isocyanateuréthanne fluoré (IV). Le polymère acrylique à groupes OH pendants peut lui-même être obtenu par polymérisation en milieu solvant dans des conditions analogues à celles précédemment décrites pour la polymérisation des monomères de formule (II).

Quelque soit leur mode d'obtention, les polymères fluorés selon l'invention peuvent éventuellement être isolés suivant des méthodes connues en soi telles que, par examples, précipitation ou évaporation du solvant.

Les polymères fluorés selon l'invention se révèlent d'excellents agents hydrophobes et oléophobes sur des matériaux trés divers tels que le papier, les articles non tissés, les textiles à base de fibres naturelles, artificielles ou synthétiques, les matières plastiques, le bois, les métaux, le verre, la pierre et le ciment, mais sont plus particulièrement destinés à la protection des cuirs qu'il s'agisse de leur finition ou de l'entretien des articles en cuir tels que vêtements, chaussures, maroquinerie, sièges ...

Pour l'application, les solutions de polymères sont généralement diluées avec un solvant identique à ou compatible avec celui utilisé pour la polymérisation, tandis que les émulsions de polymères sont diluées à l'eau. L'application des produits dilués peut être réalisée selon différentes techniques comme la pulvérisation, l'enduction à la brosse, le foulardage. Selon leur nature, les substrats traités peuvent être séchés à température ambiante ou à des températures pouvant aller jusqu'à 200°C.

La quantité de polymère à mettre en oeuvre peut varier dans de larges limites en fonction de la nature du support et de la teneur en fluor du polymère. Sur cuir, cette quantité est généralement comprise entre 1 et 10 g/m$^2$.

Les exemples suivants dans lesquels les parties et les pourcentages s'entendent en poids, sauf mention contraire, illustrent l'invention sans la limiter.

EXEMPLE 1

Dans un réacteur d'une capacité de 1000 parties en volume, équipé d'un agitateur, d'un thermométre, d'un réfrigérant à reflux, d'une ampoule de coulée, d'une arrivée d'azote et d'un dispositif de chauffage, on charge 90 parties de trichlorotrifluoroéthane et 8,7 parties de toluènediisocyanate-2,4 pur (0,05 mole). On chasse l'air du réacteur par un courant d'azote anhydre, puis on porte la solution au reflux (soit 50°C) et on coule ensuite goutte à goutte en deux heures une solution de 18,2 parties de perfluorohexyl-2 éthanol $C_6F_{13}C_2H_4OH$ (0,05 mole) et 0,1 partie de dibutyl dilaurate d'étain dans 20 parties de trichlorotrifluoroéthane. La fine suspension blanche ainsi obtenue est encore maintenue à 50°C pendant 1/2 heure. L'analyse chromatographique (G.P.C.) montre alors la disparition totale de l'alcool fluoré et la formation, à coté de l'isocyanate-2 uréthanne-4, du produit de diaddition symétrique en -2,4 (proportion molaire : 20 %).

On ajoute ensuite goutte à goutte une solution de 6,5 parties de méthacrylate d'hydroxy-2 éthyle (0,05 mole) dans 10 parties de trichlorotrifluoroéthane, et on maintient le reflux pendant une heure. Aprés évaporation du solvant, on obtient 33 g d'un mélange de monomère diuréthanne et de produit de diaddition symétrique que l'on élimine par cristallisation fractionnée dans le toluène. On obtient un liquide sirupeux incolore dont l'analyse R.M.N ($^1H$ et $^{13}C$) confirme la structure attendue :

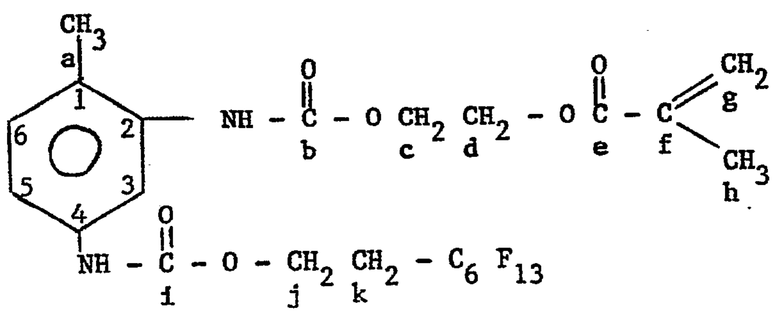

Spectre $^1H$. On observe les pics suivants:
1,95 ppm: protons du $CH_3$ en h
2,18 ppm: protons du $CH_3$ en a
2,50 ppm: protons du $CH_2$ en k
4,41 ppm: protons des $CH_2$ en c et d
4,44 ppm: protons du $CH_2$ en j
5,59 et 6,15 ppm: protons du $CH_2$ en g
6,60 ppm: proton du NH fixé en 4
7,00 ppm: proton du NH fixé en 2
7,77 ppm: proton du CH en 3

Spectre $^{13}C$. On observe les pics suivants:
16,48 ppm: Ca
17,72 ppm: Ch
30,64 ppm: Ck
56,74 ppm: Cj
62,5 et 62,9 ppm: Cc et Cd
112,85 ppm: C3
115,24 ppm: C5
123,63 ppm: C1
125,63 ppm: Cg
130,44 ppm: C6
135,8 et 135,9 ppm: C2 et Cf
136,4 ppm: C4
153,06 et 153,65 ppm: Cb et Ci
167,03: Ce

EXEMPLE 2

Dans un réacteur d'une capacité de 500 parties en volume, équipé d'un agitateur, d'un thermomètre, d'un réfrigérant à reflux, d'une ampoule de coulée, d'une arrivée d'azote et d'un dispositif de chauffage on charge 127 parties de méthyl isobutyl cétone anhydre et 17,4 parties (0,1 mole) de toluène-diisocyanate (mélange à 80 % d'isomère-2,4 et 20 % d'isomère-2,6) et 0,1 partie de dibutyldilaurate d'étain. On chasse l'air du réacteur par un courant d'azote anhydre, puis on porte la température du milieu réactionnel à 80°C à l'aide d'un bain d'huile thermostaté et on coule ensuite goutte à goutte en une heure et demie une solution, préalablement étêtée, comprenant 36,4 parties (0,1 mole) de perfluorohexyl-2 éthanol $C_6F_{13}C_2H_4OH$ et 36,4 parties de méthyl isobutyl cétone. Aprés avoir encore maintenu à 80°C pendant 30 minutes, un dosage chimique montre que la moitié des groupements -NCO a effectivement réagi. Une analyse chromatographique GPC indique la formation, à côté de l'isocyanate-2 uréthanne-4, d'une proportion molaire d'environ 20 % de produit de diaddition symétrique en -2,4.

On ajoute ensuite 0,06 partie d'éther méthylique de l'hydroquinone, puis on coule goutte à goutte en 15 minutes 13 parties (0,1 mole) de méthacrylate d'hydroxy-2 éthyle et maintient ensuite à 80°C pendant encore une heure. L'analyse chromatographique montre qu'il ne reste plus de méthacrylate d'hydroxy-2 éthyle. La solution est alors filtrée vers 35°C, puis refroidie. On obtient ainsi une solution ($S_2$) d'un mélange de monomère selon l'invention et de diuréthanne symétrique qu'il n'est pas nécessaire de séparer. Cette solution contient 29 % de matières sèches et 10,73 % de fluor.

EXEMPLE 3

On procède comme à l'exemple 2, mais en remplaçant la solution de perfluorohexyl-éthanol par une solution de 46,4 parties de perfluorooctyl-éthanol $C_8F_{17}C_2H_4OH$ dans 46,4 parties de méthyl isobutyl cétone. Le taux de transformation en produit de diaddition est un peu plus important (environ 30 %). On obtient une solution ($S_3$) qui cristallise partiellement à froid et qui contient 29 % de matière sèche et 12,2 % de fluor.

EXEMPLE 4

On procède comme à l'exemple 2, mais en remplaçant la solution de perfluorohexyl-éthanol par une solution de 48,5 parties du sulfamido-alcool fluoré de formule $C_6F_{13}C_2H_4SO_2N(CH_3)C_2H_4OH$ dans 48,5 parties de méthylisobutyl cétone. Le taux de transformation en produit de diaddition symétrique -2,4 est de 40 %. On obtient une solution ($S_4$) qui cristallise partiellement à froid et qui contient 29 % de matière sèche et 9 % de fluor.

EXEMPLE 5

On procède comme à l'exemple 2, mais en remplaçant la solution de perfluorohexyl-éthanol par une solution de 48 parties du thiol fluoré $C_8F_{17}C_2H_4SH$ dans 48 parties de méthyl isobutyl cétone. Le taux molaire en produit de diaddition symétrique est de 24 %. On obtient une solution ($S_5$) qui cristallise partiellement à froid et qui contient 29 % de matière sèche et 11,9 % de fluor.

EXEMPLE 6

On procède, comme à l'exemple 2, mais en remplaçant le méthacrylate d'hydroxy-2 éthyle par 11,6 parties d'acrylate d'hydroxy-2 éthyle (0,1 mole). La solution ($S_6$) obtenue contient 24,6 % de matière sèche et 9,3 de fluor.

EXEMPLE 7

On procède, comme à l'exemple 2, mais en remplaçant le méthacrylate d'hydroxy-2 éthyle par 13 parties d'acrylate d'hydroxy-2 propyle (0,1 mole). La solution ($S_7$) obtenue contient 25 % de matière sèche et 9,25% de fluor.

EXEMPLE 8

On procède comme à l'exemple 2, mais en remplaçant le méthacrylate d'hydroxy-2 éthyle par14,4 parties d'acrylate d'hydroxy-4 butyle (0,1 mole). La solution ($S_8$) obtenue contient 29 % de matière sèche et 10,5 % de fluor.

EXEMPLE 9

Dans un réacteur d'une capacité de 250 parties en volume, équipé d'un agitateur, d'un thermomètre, d'un réfrigérant à reflux, d'une arrivée d'azote et d'un dispositif de chauffage, on charge 125 parties de

la solution ($S_2$), puis on effectue un balayage à l'azote en surface pendant 15 minutes et porte la température à 90°C. On ajoute alors 0,3 partie de peroxyde de lauroyle et 0,2 partie de perpivalate de t.butyle. On maintient ensuite la température à 90°C pendant 6 heures en rajoutant la même charge d'initiateurs au bout de 2 et 4 heures. Après refroidissement on obtient une solution jaune limpide ($S_{2p}$) d'un homopolymère selon l'invention. Cette solution contient 29 % de matières sèches et 10,7 % de fluor.

En procédant de la même façon avec les solutions $S_3$, $S_4$, $S_5$, $S_6$, $S_7$ et $S_8$ des exemples 3 à 8 on obtient respectivement des solutions $S_{3p}$ à $S_{8p}$, d'homopolymères selon l'invention.

On dilue chacune des solutions $S_{2p}$ à $S_{8p}$ avec de la méthyl isobutyl cétone de façon à obtenir des solutions contenant chacune 0,20 % de fluor. Ces solutions diluées sont ensuite appliquées par pulvérisation sur un cuir vachette pleine fleur tanné végétal, à raison de 200g/m² et on laisse sécher pendant une nuit à température ambiante avant de procéder aux tests suivants :

• <u>TEST R.E. (résistance à l'eau)</u> : consiste à mesurer le temps de pénétration d'une goutte d'eau déposée sur le cuir.

• <u>TEST R.H. (résistance à l'huile)</u> : consiste à mesurer le temps de pénétration d'une goutte d'huile de vaseline déposée sur le cuir.

Le tableau suivant rassemble les résultats obtenus, comparativement au même cuir non traité.

| Solution de traitement | Hydrophobie RE | Oléophobie RH |
|---|---|---|
| Néant (cuir non traité) | moins de 30 secondes | moins de 10 secondes |
| $S_{2p}$ | 7,3 heures | 45 minutes |
| $S_{3p}$ | 4 heures | plus de 30 heures |
| $S_{4p}$ | 3,5 heures | plus de 30 heures |
| $S_{5p}$ | 8 heures | plus de 30 heures |
| $S_{6p}$ | 1,75 heures | 30 minutes |
| $S_{7p}$ | 0,5 heures | 30 minutes |
| $S_{8p}$ | 1,75 heures | 5 heures |

<u>EXEMPLE 10</u>

Dans un réacteur d'une capacité de 1000 parties en volume, équipé comme celui de l'exemple 9, on charge 310,3 parties de la solution ($S_2$), 50 parties de méthylisobutylcétone et 90 parties de méthacrylate d'éthyl-2 hexyle. Aprés avoir purgé à l'azote on porte la température à 90°C, puis on ajoute 0,3 parties de peroxyde de lauroyle et 0,2 parties de perpivalate de t.butyle et la température est ensuite maintenue à 90°C pendant 6 heures en rajoutant la même quantité d'initiateurs toutes les deux heures.

La solution jaune limpide de copolymère ($S_{10}$) ainsi obtenue contient 40 % de matières non volatiles et 7,5 % de fluor.

<u>EXEMPLE 11</u>

Dans des conditions identiques à celles de l'exemple 10, on copolymérise 310,3 parties de solution ($S_2$), 30,6 parties de méthacrylate d'éthyl-2 hexyle et 59,4 parties de méthacrylate de perfluorohexyl-2 éthyle dans 50 parties de méthyl isobutyl cétone.

La solution de copolymère ainsi obtenue ($S_{11}$) est limpide et contient 39,1 % de matières non volatiles et 14,6 % de fluor.

<u>EXEMPLE 12</u>

On opère comme à l'exemple 10, mais on n'utilise que 36 parties de méthacrylate d'éthyl-2 hexyle, le reste étant remplacé par 54 parties d'un mélange de monomères acryliques polyfluorés de formule :

$$CF_3(CF_2)_n-C_2H_4-O\underset{\underset{O}{\|}}{C}-\overset{\overset{CH_3}{|}}{C}=CH_2$$

où n est égal à 5, 7, 9, 11, 13 et 15 dans des rapports en poids moyens et respectifs de 47 : 32 : 13 : 5 : 2 : 1.

La solution de copolymère ainsi obtenue ($S_{12}$) est jaune, limpide, légérement visqueuse. Elle contient 38,8 % de matières non volatiles et 14,1 % de fluor.

EXEMPLE 13

Selon le même mode opératoire qu'à l'exemple 10, on copolymérise 310,3 parties de solution ($S_2$), 16,2 parties de méthacrylate d'éthyle-2 hexyle et 73,8 parties d'un mélange d'esters acryliques fluorés de formule :

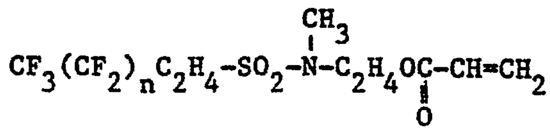

où n est égal à 3, 5, 7, 9, 11, 13 et 15 dans les rapports en poids moyens et respectifs de 1 : 50 : 31 : 10 : 3 : 1 : 1, dans 25 parties de méthyl isobutyl cétone et 25 parties d'acétone. On obtient une solution ($S_{13}$) jaune brun, limpide, contenant 40,6 % de matières non volatiles et 15,1 % de fluor.

EXEMPLE 14

En opérant comme au premier paragraphe de l'exemple 9, on copolymérise 86,2 parties de solution ($S_8$), 10 parties de méthacrylate d'éthyl-2 hexyle et 15 parties du même mélange de monomères acryliques polyfluorés qu'à l'exemple 12.

Le copolymère ($S_{14}$) ainsi obtenu se présente sous la forme d'une masse gélatineuse dont le taux de amtières sèches est de 40,1 % de le taux de fluor de 14,5 %.

EXEMPLE 15

En opérant comme à l'exemple 9, on copolymérise 69 parties de solution ($S_3$), 5 parties de méthacrylate d'éthyl-2 hexyle et 25 parties d'un mélange de monomères polyfluorés de formule :

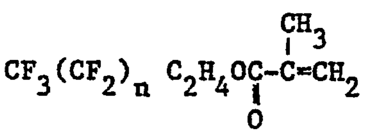

où n est égal à 5, 7, 9, 11, 13 et 15 dans les rapports en poids moyens et respectifs de 1 : 56 : 22 : 9 : 3 : 3, dans 25 parties de méthyl isobutyl cétone. Quand la polymérisation est terminée on dilue le mélange avec 125 parties de trichlorotrifluoro-éthane.

On obtient une solution ambrée limpide ($S_{15}$) contenant 18,8 % matières non volatiles et 9 % de fluor.

EXEMPLE 16

Dans un réacteur d'une capacité de 500 parties en volume, muni d'un agitateur, d'un thermomètre, d'un réfrigérant à reflux et d'une arrivée d'azote, on introduit 90 parties de méthyl isobutyl cétone, 55 parties de méthacrylate de stéaryle et 5 parties de méthacrylate d'hydroxy-2 éthyle. On chauffe le mélange sous atmosphère d'azote pendant 1h30 à 80°C en présence de 0,4 partie de peroxyde de lauroyle et 0,25 partie de perpivalate de t.butyle. Le dosage chromatographique (G.P.C) indique un taux de polymérisation de 70 %.

On ajoute alors 92,7 parties d'une solution d'isocyanateuréthanne, obtenue en opérant comme au premier paragraphe de l'exemple 2. On chauffe ensuite le mélange à 80°C pendant 4 heures en ajoutant 0,4 parties de peroxyde de lauroyle et 0,2 partie de perpivalate de t.butyle.

Le copolymère greffé ($S_{16}$) ainsi obtenu se présente sous la forme d'une solution épaisse ambrée dont le taux de matières sèches est de 37,7 % et le taux de fluor de 4,7 %.

EXEMPLE 17

17-a: Dans un réacteur identique à celui de l'exemple 16, on copolymérise 34,7 parties de méthacrylate de butyle, 39 parties du même mélange de monomères polyfluorés qu'à l'exemple 15, et 13 parties de méthacrylate d'hydroxy-2 éthyle dans 87 parties de méthyl isobutyl cétone. On opère sous atmosphère d'azote et à 100°C pendant 6 heures, en ajoutant initialement 1 partie de peroxyde de lauroyle et 0,2 partie de perpivalate de t.butyle et en renouvelant cette addition d'initiateurs aprés 2 et 4 heures.

17-b: Dans un réacteur identique à celui de l'exemple 2 on introduit 88 parties de méthyl isobutyl cétone, 17,4 parties de toluènediisocyanate (à 80 % d'isomère-2,4) et 0,1 partie de dibutyl dilaurate d'étain. Aprés avoir chassé l'air du réacteur par un courant d'azote anhydre, on porte la température à 80°C,

puis on coule goutte à goutte en une heure 36,4 parties de perfluorohexyléthanol en solution dans 36,4 parties de méthyl isobutyl cétone. On maintient à 80°C pendant encore une heure, puis on ajoute la totalité du copolymère hydroxylé synthétisé à l'étape 17-a et on porte à 100°C pendant 4 heures.

On obtient ainsi une solution assez visqueuse ($S_{17}$) qui contient 39,7 % de matières non volatiles et 13,8 % de fluor.

EXEMPLE 18

On procède comme à l'exemple 2 pour faire réagir le perfluorohexyl-éthanol $C_6F_{13}C_2H_4OH$ sur le toluène diisocyanate. Puis on ajoute goutte à goutte en 1/4 heure à 80°C un mélange de 6,5 parties de méthacrylate d'hydroxy-2 éthyle (0,05 mole) et 9,25 parties de méthacrylate de t.butylamino-2 éthyle (0,05 mole).

Le mélange de monomères diuréthanne et uréthanne-urée ainsi obtenu est copolymérisé à 90°C en présence de 0,5 partie de peroxyde de lauroyle et 0,4 partie de perpivalate de t.butyle ajoutés toutes les deux heures pendant 6 heures.

On obtient une solution ($S_{18}$) jaune brun contenant 29,1 % de matières séches et 10,35 % de fluor.

EXEMPLE 19

On dilue les solutions $S_{10}$, $S_{11}$, $S_{12}$, $S_{13}$, $S_{14}$, $S_{15}$, $S_{16}$, $S_{17}$ et $S_{18}$ des exemples précédents avec de la méthyl isobutyl cétone de façon à obtenir des solutions $S_{10d}$ à $S_{18d}$ contenant 0,4 % de fluor. Ces solutions diluées sont ensuite appliquées par pulvérisation sur un cuir "vachette pleine fleur-tanné végétal" à raison de 200 g/m² et on laisse sécher pendant une nuit à température ambiante avant de procéder aux mêmes tests qu'à l'exemple 9. Les résultats obtenus sont rassemblés dans le tableau suivant :

| | Hydrophobie RE | Oléophobie RH |
|---|---|---|
| Cuir non traité | moins de 30 secondes | moins de 10 secondes |
| Cuir traité avec: | | |
| $S_{10d}$ | 6,5 heures | plus de 30 heures |
| $S_{11d}$ | 6,5 heures | plus de 30 heures |
| $S_{12d}$ | 7,5 heures | plus de 30 heures |
| $S_{13d}$ | 8 heures | plus de 30 heures |
| $S_{14d}$ | 9 heures | plus de 30 heures |
| $S_{15d}$ | 4,5 heures | plus de 30 heures |
| $S_{16d}$ | 7,75 heures | plus de 30 heures |
| $S_{17d}$ | plus de 9 heures | plus de 30 heures |
| $S_{18d}$ | plus de 9 heures | plus de 30 heures |

EXEMPLE 20

Dans un réacteur identique à celui de l'exemple 1, on charge 220 parties d'acétate de butyle préalablement étété, 34,8 parties (0,2 mole) de toluène-diisocyanate (mélange à 80 % d'isomère-2,4 et 20 % d'isomère-2,6) et 0,2 partie de dibutyl dilaurate d'étain. On chasse l'air du réacteur par un courant d'azote anhydre, puis porte la température à 80°C à l'aide d'un bain d'huile thermostaté et coule ensuite en deux heures une solution comprenant 72,8 parties (0,2 mole) de perfluorohexyl-2 éthanol $C_6F_{13}C_2H_4OH$ et 72,8 parties d'acétate de butyle anhydre. On ajoute ensuite 0,12 partie d'éther méthylique de l'hydroquinone, puis on coule en environ 15 minutes 26 parties (0,2 mole) de méthacrylate d'hydroxy-2 éthyle et 26 parties d'acétate de butyle anhydre. Aprés 1 heure à 80°C on ajoute à la solution ainsi obtenue, 35,6 parties de méthacrylate d'éthyl-2 hexyle, 8,9 parties de méthacrylate d'hydroxy-2 éthyle et 100 parties d'acétate de butyle. La température est portée à 90°C, et on introduit 1 partie de peroxyde de lauroyle et 0,7 partie de perpivalate de t.butyle. Au bout de 3 heures la polymérisation est terminée et on obtient un terpolymère solon l'invention, qui se présente sous la forme d'une solution jaune clair ($S_{20}$) limpide contenant 30 % de matière sèche et 8,3 % de fluor.

La solution ($S_{20}$) est diluée par l'isopropanol de façon à obtenir une solution contenant 0,4 % de fluor, qui est appliquée dans les conditions décrites à l'exemple 19. Les résultats sont les suivants :
RE : supérieur à 9 heures
RH : supérieur à 30 heures

## Revendications

1. Monomères acryliques fluorés, caractérisés en ce qu'ils répondent à la formule générale:

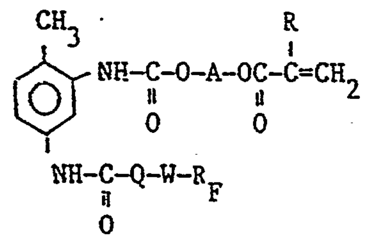

(II)

dans laquelle
$R_F$ représente un radical perfluoroalkyle à chaîne droite ou ramifiée contenant de 2 à 20 atomes de carbone,
R représente un atome d'hydrogène ou un radical méthyle,
A représente un enchaînement bivalent de 2 à 9 atomes de carbone, pouvant comporter un ou plusieurs atomes d'oxygène,
W–Q représente un enchaînement bivalent choisi parmi ceux de formules:

$$-(CH_2)_p-O-$$
$$-SO_2N(R')-(CH_2)_q-O-$$
$$-(CH_2)_p-SO_2N(R')-(CH_2)_q-O-$$
$$-(CH_2)_p-O-(CH_2)_q-O-$$
$$-(CH_2)_p-S-(CH_2)_q-O-$$
$$-(CH_2)_p-(OCH_2CH_2)_q-O-$$
$$-(CH_2)_p-SO_2-(CH_2)_q-O-$$
$$-C(=O)-N(R')-(CH_2)_p-O-$$
$$-C(=O)-O-(CH_2)_p-O-$$
$$-CH=CH-(CH_2)_p-O-$$
$$-(CH_2)_pS-$$
$$-(CH_2)_pNH-$$
$$-SO_2-N(R')-(CH_2)_qNH-$$
$$-SO_2-N(R')-(CH_2)_q-N(CH_3)-$$

R' désigne un atome d'hydrogène ou un radical alkyle contenant 1 à 4 atomes de carbone,
p et q, identiques ou différents, représentent chacun un nombre entier allant de 1 à 20.

2. Monomères selon la revendication 1, dans lesquels le radical perfluoroalkyle $R_F$ contient de 4 à 16 atomes de carbone, R est un radical méthyle, A est un pont $-CH_2CH_2-$, et –Q–W– est un enchaînement $-O-CH_2CH_2-$, $-S-CH_2CH_2-$ ou $-O-CH_2CH_2N(R')SO_2CH_2CH_2-$, R′ représentant un atome d'hydrogène ou un radical méthyle.

3. Procédé de préparation de monomères selon la revendication 1, caractérisé en ce qu'on fait réagir dans un premier temps le toluènediisocyanate-2,4 avec une quantité sensiblement équimolaire d'un composé polyfluoré de formule:

$R_F - W - Q - H$ (III)

pour former un isocyanate-uréthanne fluoré de formule:

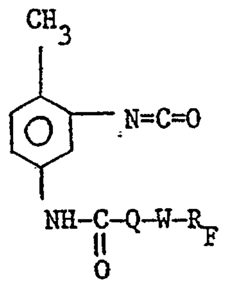

(IV)

que l'on fait réagir ensuite avec une quantité sensiblement équimolaire d'un ester acrylique de formule:

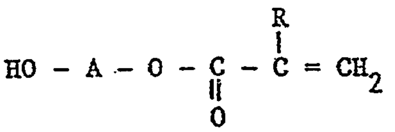

(V)

les symboles A, R, $R_F$ et W–Q ayant les mêmes significations que dans la revendication 1.

4. Procédé selon la revendication 3, caractérisé en ce que l'on opère sous atmosphère inerte, à une température allant de 30 à 90°C et dans un solvant organique inerte.

5. Procédé selon la revendication 3 ou 4 , caractérisé en ce que le composé polyfluoré correspond à l'une des formules suivantes:

$R_F - CH_2\,CH_2\,OH$
$R_F - CH_2\,CH_2\,SO_2\,N(R') - CH_2\,CH_2\,OH$
$R_F - CH_2\,CH_2\,SH$

dans lesquelles $R_F$ contient de 4 à 16 atomes de carbone et R′ est un atome d'hydrogène ou un radical méthyle.

6. Procédé selon l'une des revendications 3 à 5, caractérisé en ce que l'ester (V) est le méthacrylate d'hydroxy-2 éthyle.

7. Polymères contenant de 10 à 100% en poids de motifs de formule générale:

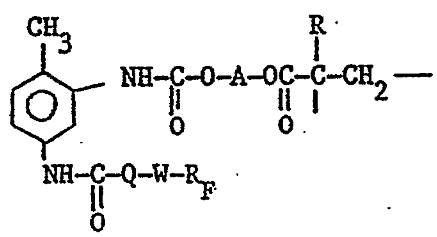

(VIII)

obtenus par homopolymérisation des monomères selon la revendication 1, par copolymérisation desdits monomères avec d'autres monomères fluorés ou non, ou par greffage d'un isocyanate-uréthanne fluoré de formule:

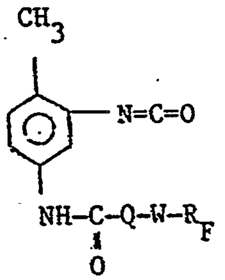

(IV)

sur un homopolymère d'un ester acrylique de formule:

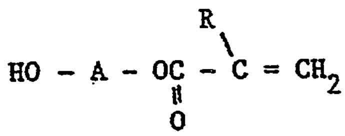

(V)

ou un copolymère d'un tel ester avec d'autres monomères fluorés ou non, les symboles $R_F$, R, A et W–Q ayant les mêmes significations que dans la revendication 1.

8. Copolymères selon la revendication 7, dans lesquels le ou les comonomères sont choisis parmi les acrylates ou méthacrylates d'alkyle, les composés de formule:

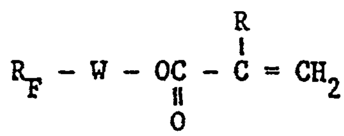

et leurs mélanges,
W représente un enchaînement bivalent choisi parmi ceux de formules:

$-(CH_2)_p-$

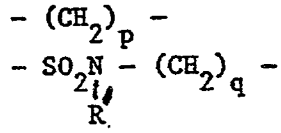

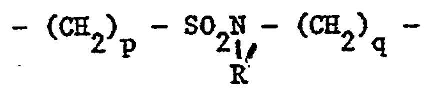

$-(CH_2)_p-O-(CH_2)_q-$

$-(CH_2)_p-S-(CH_2)_q-$

$-(CH_2)_p-(OCH_2CH_2)_q-$

$-(CH_2)_p-SO_2-(CH_2)_q-$

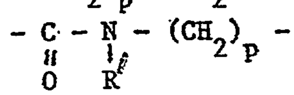

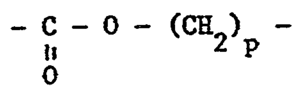

$-CH=CH-(CH_2)_p-$

les symboles $R_F$, R, R', p et q ayant les mêmes significations que dans la revendication 1.

9. Application des polymères selon la revendication 7 ou 8 à l'oléofugation et à l'hydrofugation de substrats divers, en particulier des cuirs.

10. Matériaux et articles traités au moyen d'un polymère selon la revendication 7 ou 8.

## Patentansprüche

1. Fluorierte acrylische Monomere, dadurch gekennzeichnet, daß sie der allgemeinen Formel

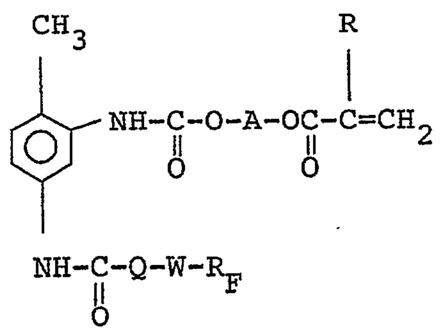

(II)

entsprechen, in der
$R_F$ einen Perfluoralkylrest mit gerader oder verzweigter Kette darstellt, der 2 bis 20 Kohlenstoffatome enthält,
R ein Wasserstoffatom oder einen Methylrest darstellt,
A eine bivalente Kette mit 2 bis 9 Kohlenstoffatomen dargestellt, die 1 oder mehrere Sauerstoffatome enthalten kann,
W–Q eine bivalente Kette darstellt, ausgewählt aus denjenigen der Formeln:

$-(CH_2)_p-O-$

$-SO_2N(R')-(CH_2)_q-O-$

$-(CH_2)_p-SO_2N(R')-(CH_2)_q-O-$

$-(CH_2)_p-O-(CH_2)_q-O-$

$-(CH_2)_p-S-(CH_2)_q-O-$

$-(CH_2)_p-(OCH_2CH_2)_q-O-$

$-(CH_2)_p-SO_2-(CH_2)_q-O-$

$-C(=O)-N(R')-(CH_2)_p-O-$

$-C(=O)-O-(CH_2)_p-O-$

$-CH=CH-(CH_2)_p-O-$

$-(CH_2)_pS-$

$-(CH_2)_pNH-$

$-SO_2-N(R')-(CH_2)_qNH-$

$-SO_2-N(R')-(CH_2)_q-N(CH_3)-$

wobei R′ ein Wasserstoffatom oder einen Alkylrest mit 1 bis 4 Kohlenstoffatomen darstellt, p und q identisch oder verschieden sind und jeweils eine ganze Zahl von 1 bis 20 darstellen.

2. Monomere gemäß Anspruch 1, bei denen der Perfluoralkylrest $R_F$ 4 bis 16 Kohlenstoffatome enthält, R ein Methylrest ist, A eine Brücke $-CH_2-CH_2-$ ist und $-Q-W-$ eine Kette $-O-CH_2-CH_2-$, $-S-CH_2CH_2-$ oder $-O-CH_2CH_2N(R')SO_2CH_2CH_2-$ ist, in der R′ ein Wasserstoffatom oder einen Methylrest darstellt.

3. Verfahren zur Herstellung von Monomeren gemäß Anspruch 1, dadurch gekennzeichnet, daß man zunächst das 2,4-Toluoldiisocyanat mit einer im wesentlichen äquimolaren Menge einer polyfluorierten Verbindung der Formel:

$R_F - W - Q - H$ (III)

reagieren läßt, um ein fluoriertes Isocyanat-Urethan der Formel:

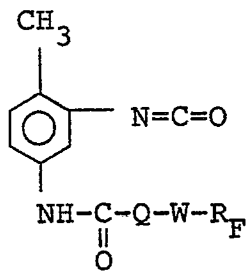

(IV)

zu bilden, das man anschließend mit einer im wesentlichen äquimolaren Menge eines acrylischen Esters der Formel:

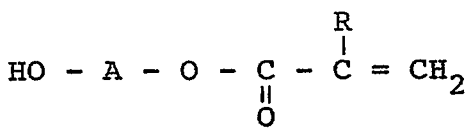

(V)

reagieren läßt in der die Symbole A, R, $R_F$ und W–Q die gleiche Bedeutung wie in Anspruch 1 haben.

4. Verfahren nach Anspruch 3, dadurch gekennzeichnet, daß man unter Inertatmosphäre bei einer Temperatur von 30 bis 90°C und in einem inerten organischen Lösungsmittel arbeitet.

5. Verfahren nach Anspruch 3 oder 4, daduch gekennzeichnet, daß die polyfluorierte Verbindung einer der nachfolgenden Formeln entspricht:

$R_F - CH_2 CH_2 OH$
$R_F - CH_2 CH_2 SO_2 N(R') - CH_2 CH_2 OH$
$R_F - CH_2 CH_2 SH$

in denen $R_F$ 4 bis 16 Kohlenstoffatome enthält und R′ ein Wasserstoffatom oder ein Methylrest ist.

6. Verfahren nach einem der Ansprüche 3 bis 5, dadurch gekennzeichnet, daß der Ester (V) das 2-Hydroxyethylmethacrylat ist.

7. Polymere enthaltend 10 bis 100 Gew.-% an Baugruppen der allgemeinen Formel

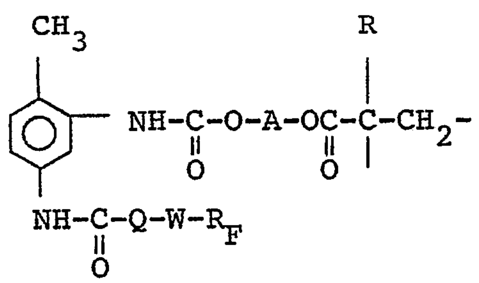

(VIII)

erhalten durch Homopolymerisation von Monomeren gemäß Anspruch 1, durch Copolymerisation dieser Monomeren mit anderen fluorierten oder nichtfluorierten Monomeren oder durch Aufpfropfung eines fluorierten Isocyanat-Urethans der Formel:

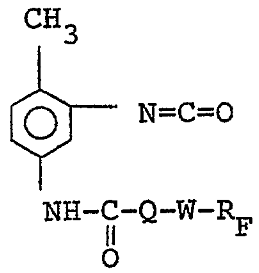

(IV)

auf ein Homopolymer eines acrylischen Esters der Formel:

$$HO - A - OC(=O) - C(R) = CH_2 \quad (V)$$

oder ein Copolymer eines solchen Esters mit anderen fluorierten oder nicht fluorierten Monomeren, wobei die Symbole $R_F$, R, A und W–Q die gleiche Bedeutung haben wie in Anspruch 1.

8. Copolymere gemäß Anspruch 7, bei denen das oder die Comonomeren ausgewählt werden aus den Alkylacrylaten oder -methacrylaten, den Verbindungen der Formel:

$$R_F - W - OC(=O) - C(R) = CH_2$$

und deren Gemischen, wobei W eine bivalente Kette ausgewählt aus denjenigen der Formeln:

$$-(CH_2)_p-$$
$$-SO_2N(R')-(CH_2)_q-$$
$$-(CH_2)_p-SO_2N(R')-(CH_2)_q-$$
$$-(CH_2)_p-O-(CH_2)_q-$$
$$-(CH_2)_p-S-(CH_2)_q-$$
$$-(CH_2)_p-(OCH_2CH_2)_q-$$
$$-(CH_2)_p-SO_2-(CH_2)_q-$$
$$-C(=O)-N(R')-(CH_2)_p-$$
$$-C(=O)-O-(CH_2)_p-$$
$$-CH=CH-(CH_2)_p-$$

darstellt, worin die Symbole $R_F$, R, R', p und q die gleiche Bedeutung haben wie in Anspruch 1.

9. Verwendung der Polymere gemäß Anspruch 7 oder 8, zum ölabweisend und wasserabweisend Machen verschiedener Substrate, insbesondere von Leder.

10. Materialien und Artikel die mittels eines Polymeren gemäß Anspruch 7 oder 8 behandelt wurden.

**Claims**

1. Fluorine-containing acrylic monomers characterized in that they correspond to the general formula:

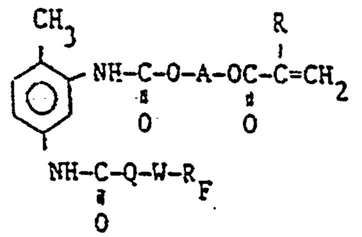

(II)

in which

$R_F$ denotes a perfluoroalkyl radical with a straight or branched chain containing from 2 to 20 carbon atoms,
R denotes a hydrogen atom or a methyl radical,
A denotes a divalent chain sequence of 2 to 9 carbon atoms,
which may comprise one or more oxygen atoms,
W–Q denotes a divalent chain sequence chosen from those of formulae:

$$-(CH_2)_p-O-$$

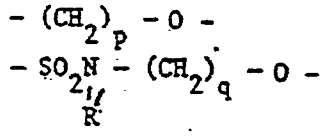

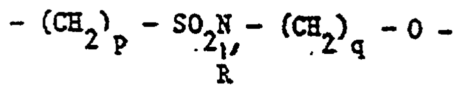

$$-(CH_2)_p-O-(CH_2)_q-O-$$
$$-(CH_2)_p-S-(CH_2)_q-O-$$
$$-(CH_2)_p-(OCH_2CH_2)_q-O-$$
$$-(CH_2)_p-SO_2-(CH_2)_q-O-$$

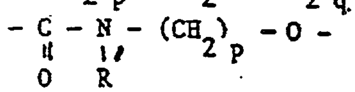

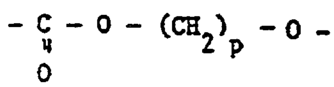

$$-CH=CH-(CH_2)_p-O-$$

$$-(CH_2)_pS-$$
$$-(CH_2)_pNH-$$

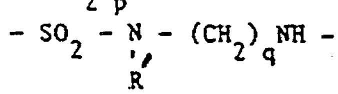

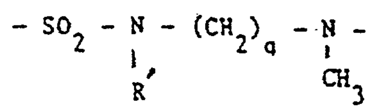

R′ denotes a hydrogen atom or an alkyl radical containing 1 to 4 carbon atoms,
each of p and q, which are identical or different, denotes an integer ranging from 1 to 20.

2. Monomers according to claim 1, in which the perfluoroalkyl radical $R_F$ contains from 4 to 16 carbon atoms, R is a methyl radical, A is a $-CH_2CH_2-$ bridge, and –Q–W– is a $-O-H_2CH_2-$, $-S-CH_2CH_2-$ or $-O-CH_2CH_2N(R')SO_2CH_2CH_2-$ chain sequence, R′ denoting a hydrogen atom or a methyl radical.

3. Process for the preparation of monomers according to claim 1, characterized in that in a first stage 2,4-toluene diisocyanate is reacted with a substantially equimolar quantity of a polyfluoro compound of formula:

$R_F$–W–Q–H (III)

to form a fluorine-containing urethane-isocyanate of formula:

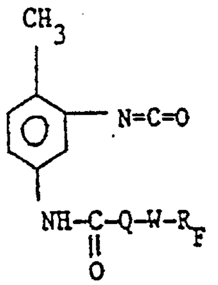

(IV)

which is then reacted with a substantially equimolar quantity of an acrylic ester of formula:

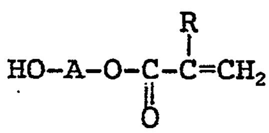

(V)

the symbols A, R, $R_F$ and W–Q having the same meanings as in claim 1.

4. Process according to claim 3, characterized in that the operation is carried out under inert atmosphere, at a temperature ranging from 30 to 90°C and in an inert organic solvent.

5. Process according to claim 3 or 4, characterized in that the polyfluoro compound corresponds to one of the following formulae:

$R_F$–$CH_2CH_2OH$
$R_F$–$CH_2CH_2SO_2N(R')$–$CH_2CH_2OH$
$R_F$–$CH_2CH_2SH$

in which $R_F$ contains 4 to 16 carbon atoms and R′ is a hydrogen atom or a methyl radical.

6. Process according to one of claims 3 to 5, characterized in that the ester (V) is 2-hydroxyethyl methacrylate.

7. Polymers containing from 10 to 100% by weight of units of general formula:

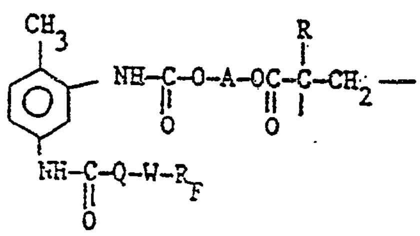

(VIII)

which are obtained by homopolymerization of the monomers according to claim 1, by copolymerization of the said monomers with other monomers, fluorine-containing or otherwise, or by grafting a fluorine-containing urethane-isocyanate of formula:

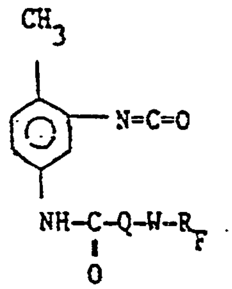

(IV)

onto a homopolymer of an acrylic ester of formula:

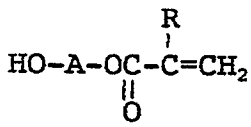

(V)

or a copolymer of an ester of this kind with other monomers, fluorine-containing or otherwise, the symbols $R_F$, R, A and W–Q having the same meanings as in claim 1.

8. Copolymers according to claim 7, in which the comonomer(s) is (are) chosen from alkyl acrylates or methacrylates, compounds of formula:

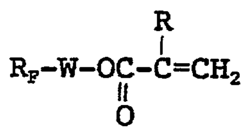

and mixtures thereof,
W denotes a divalent chain sequence chosen from those of formulae:

$$-(CH_2)_p-$$

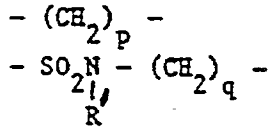

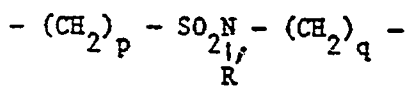

$$-(CH_2)_p-O-(CH_2)_q-$$

$$-(CH_2)_p-S-(CH_2)_q-$$

$$-(CH_2)_p-(OCH_2CH_2)_q-$$

$$-(CH_2)_p-SO_2-(CH_2)_q-$$

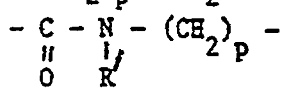

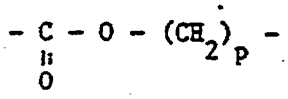

$$-CH=CH-(CH_2)_p-$$

the symbols $R_F$, R, R′, p and q having the same meanings as in claim 1.

9. Application of the polymers according to claim 7 or 8 to the oilproofing and to the waterproofing of various substrates, in particular leathers.

10. Materials and articles treated by means of a polymer according to claim 7 or 8.